# EUROPEAN PATENT APPLICATION

(11) **EP 4 268 943 A1**
(43) Date of publication of application: **01.11.2023**
(21) Application number: 23167829.3
(22) Date of filing: 13.04.2023
(51) Int. Cl.: B01D 61/14, C07K 1/34, B01D 65/08, B01D 61/08, B01D 61/18, B01D 61/28, B01D 61/10, B01D 61/20, B01D 61/30, B01D 61/12, B01D 61/22, B01D 61/32

(54) **A DEVICE FOR TANGENTIAL FLOW FILTRATION OF A FLUID**

(30) Priority: 27.04.2022 US 202263335436 P
(71) Applicant: Levitronix GmbH, 8048 Zürich (CH)
(72) Inventor: Hahn, Jürgen, Marlboroug, 07152 (US); Schöb, Reto, 8832 Wollerau (CH); Sibilia, Antony, 8157 Dielsdorf (CH); Stöckli, Simon, 8305 Dietlikon (CH)
(74) Representative: IPS Irsch AG

(57) **Abstract**

A device for tangential flow filtration of a fluid is proposed, comprising a filter unit (2), which has a first fluid opening (21) and a second fluid opening (22) for the fluid, as well as a filter element (25) and a permeate opening (23) for discharging a permeate filtered out of the fluid, wherein the device further comprises a first flow connection (31) by means of which the first fluid opening (21) can be connected to a reservoir (8) for the fluid, and a second flow connection (32) by means of which the second fluid opening (22) can be connected to the reservoir (8) for the fluid, wherein a first pump (41) is provided in the first flow connection (31), with which the fluid can be conveyed from the reservoir (8) to the filter unit (2). A third flow connection (33) is provided, configured for a fluid communication between the first flow connection (31) and the second flow connection (32), wherein the third flow connection (33) bypasses the filter unit (2), and wherein a second pump (42) is provided for circulating the fluid through the third flow connection (33) and the filter unit (2). Furthermore, a method for tangential flow filtration is proposed.

## Description

The invention relates to a device and a method for tangential flow filtration of a fluid according to the preamble of the independent patent claim.

Tangential flow filtration, which is also referred to as cross-flow filtration, is a well-known method of filtering fluids that is used, for example, in the biotechnology and chemical industries, as well as in the food and pharmaceutical industries. In tangential flow filtration, the fluid to be filtered, for example a suspension, is guided parallel to a filter element, e.g., a filter membrane, at a flow velocity different from zero, and the permeate, also designated as filtrate, is removed transverse to the flow direction. Due to a relatively high flow velocity, it should be avoided as far as possible that a filter cake or a cover layer builds up on the filter element.

Tangential flow filtration is often performed as a batch process in which the fluid to be filtered, for example a suspension, is taken from a reservoir, conveyed by means of a conveying pump through the filter unit with the filter element arranged therein, and then guided back from the filter unit to the reservoir. The permeate, which passes through the filter element in the filter unit transverse to the flow direction, is discharged or removed through a permeate opening. Flexible tubes or flexible tube connections are often selected for the flow connections between the reservoir and the filter unit.

In such processes, tangential flow filtration can be used to concentrate a fluid, e.g., a suspension, by circulating the suspension several times and in each case filtering out a liquid component, e.g., water or a culture medium, which is removed in each case as permeate, so that the suspension in the reservoir becomes increasingly concentrated. However, it is also possible to remove the substance to be recovered by tangential flow filtration, for example a protein, as permeate.

In particular, it is also known to use tangential flow filtration in combination with bioreactors, both in continuous processes and in batch processes, for example in processes for culturing cells or other biological material. For example, perfusion processes with bioreactors are known that are used for the continuous cultivation of cells, whereby, for example, metabolic products of the cells are separated out by means of tangential flow filtration and the cells are guided back to the bioreactor. For example, a culture medium for the cells can be continuously fed to the bioreactor, thereby replacing the mass or volume of the filtered-out components. The fluid in the bioreactor is also referred to as cell broth. In particular also for such applications in perfusion bioreactors, there is an increasing tendency today to design components of the device as single-use parts in order to avoid or reduce to a minimum time-consuming sterilization processes.

Conventional methods and devices for filtering cell culture or other components from a perfusion bioreactor have several disadvantages. One of the problems is filter fouling and the deposition of components at or in the filter element. To address this problem, it is a known measure to occasionally reverse the flow through the filter unit to remove depositions from the filter element.

In EP 4 101 520 A1 a device for tangential flow filtration of a fluid is disclosed, comprising a filter unit, which has a first fluid opening and a second fluid opening for the fluid, as well as a filter element and a permeate opening for discharging a permeate filtered out of the fluid. The device further comprises a first flow connection by means of which the first fluid opening can be connected to a reservoir for the fluid, and a second flow connection by means of which the second fluid opening can be connected to the reservoir for the fluid. A first centrifugal pump is provided in the first flow connection, with which the fluid can be conveyed from the reservoir to the filter unit, wherein a first control unit is provided for actuating the first centrifugal pump. The filter unit is designed in such a way that the fluid for tangential flow filtration in the filter unit can flow substantially parallel to the filter element. A second centrifugal pump for the fluid is provided in the second flow connection, by means of which a counter-pressure can be generated at the second fluid opening, wherein a second control unit is provided for actuating the second centrifugal pump.

With said device the tangential flow filtration can be performed in an alternating mode comprising a first operating mode in which the fluid flows from the first fluid opening to the second fluid opening and a second operating mode, in which the fluid flows from the second fluid opening to the first fluid opening. By changing the flow direction with this operation in alternating mode the filter element is alternately overflowed in opposite directions, whereby deposits on the filter element or the build-up of an undesired filter cake on the filter element can be avoided more efficiently for many applications.

The changing between the first operating mode and the second operating mode can be performed according to a predeterminable time scheme. It is possible, for example, to perform the first operating mode and the second operating mode in a 50% to 50% ratio, but of course other ratios such as 75% to 25% are also possible.

In EP 3 047 013 B1 the following method for processing a cell culture comprising steps (a) to (f) is proposed:
(a) providing an open circuit filtration system comprising a bioreactor comprising a cell culture, a tangential flow filtration (TFF) unit having first and second inlets, a first conduit in fluid communication between the bioreactor and the TFF unit first inlet, and a second conduit in fluid communication between the bioreactor and the TFF unit second inlet, and at least one pump disposed within the system for flowing fluid through the system, wherein the system is configured such that fluid can be flowed reversibly through the system from or to the bioreactor and through the first and second conduits and the TFF unit via the at least one pump, and filtrate can be collected from the TFF unit;
(b) flowing cell culture from the bioreactor through the first and second conduits and the TFF unit in a first flow direction for a first period of time,
(c) reversing the first flow direction and flowing the cell culture through the first and second conduits and the TFF unit in a second flow direction for a second period of time;
(d) reversing the second flow direction and flowing the culture through the first and second conduits and the TFF unit in the first flow direction for a third period of time;
(e) repeating steps (c) - (d) at least two times; and
(f) collecting the filtrate.

Thus, according to EP 3 047 013 it is also proposed to change the flow direction of the fluid through the filtration unit and through the entire system. When the fluid flows in the first flow direction through the system, the fluid flows from the bioreactor through the first conduit to the first inlet of the TFF unit, leaves the TFF unit through the second inlet of the TFF unit and is returned to the bioreactor through the second conduit. When the flow direction of the fluid is reversed, the fluid flows in the second flow direction through the system, i.e. the fluid flows from the bioreactor through the second conduit to the second inlet of the TFF unit, leaves the TFF unit through the first inlet of the TFF unit and is returned to the bioreactor through the first conduit

Starting from this state of the art, it is therefore an object of the invention to propose a different device and a different method for tangential flow filtration of a fluid, in which the fouling of the filter element can be avoided.

The subject matters of the invention meeting this object are characterized by the features of the independent patent claim of the respective category.

Thus, according to the invention, a device for tangential flow filtration of a fluid is proposed, comprising a filter unit, which has a first fluid opening and a second fluid opening for the fluid, as well as a filter element and a permeate opening for discharging a permeate filtered out of the fluid, wherein the device further comprises a first flow connection by means of which the first fluid opening can be connected to a reservoir for the fluid, and a second flow connection by means of which the second fluid opening can be connected to the reservoir for the fluid, wherein a first pump is provided in the first flow connection, with which the fluid can be conveyed from the reservoir to the filter unit. A third flow connection is provided, configured for a fluid communication between the first flow connection and the second flow connection, wherein the third flow connection bypasses the filter unit, and wherein a second pump is provided for circulating the fluid through the third flow connection and the filter unit.

The device for tangential flow filtration of a fluid is configured to be operated in two different modes. In the first operational mode, which corresponds to the usual filtration process, the first pump circulates the fluid from the reservoir, e.g. a bioreactor or a perfusion bioreactor, through the first flow connection to the filter unit and the retentate is recirculated from the filter unit to the reservoir. The fluid is moved through the filter unit in a first flow direction from the first fluid opening to the second fluid opening of the filter unit.

The second operational mode is a purging mode for purging the filter element. The second operational mode can act as purge and filtration mode at the same time. In the second operational mode the fluid is moved through the filter unit in a second flow direction from the second fluid opening to the first fluid opening of the filter unit. Thus, in the second operational mode, the direction of the flow through the filter unit is reversed as compared to the first operational mode. This causes the purging of the filter element.

After the filter element has been sufficiently purged, the device may be switched back to the first operational mode.

At least in the second operational mode a partial flow of the fluid passes through the third flow connection. This partial flow is conveyed by the second pump from the second fluid opening to the first fluid opening of the filter unit into the first flow connection and from there through the third flow connection into the second flow connection. Hence, this partial flow for purging the filter element is circulated through the third flow connection and the filter unit. Therefore, in the second operational mode at least a part of the fluid leaving the filter unit is not recycled to the reservoir but passes through the third flow connection and is directly recycled to the filter unit.

Thus, in the second operational mode, at least a part of the fluid leaving the filter unit bypasses the reservoir and is directly recycled to the filter unit.

Preferably the device comprises a first branching point arranged at the first flow connection, and a second branching point arranged at the second flow connection, wherein the third flow connections extends from the first branching point to the second branching point, and wherein the first branching point is arranged between the first pump and the first fluid opening of the filter unit.

In some embodiments a first flow controller is provided for opening or closing the passage through the third flow connection. The first flow controller can be configured, for example, as a valve or a clamp, e.g. as a shut-off valve or as a pinch valve. By means of the first flow controller the third flow connection can be closed during the first operational mode, so that the fluid cannot pass through the third flow connection. The fluid leaving the filter unit is completely recycled to the reservoir. During the second operational mode, the first fluid controller opens the passage through the third flow connection, so that at least a part of the fluid discharged from the filter unit is directly recycled to the filter unit, i.e. bypassing the reservoir.

In some embodiments a second flow controller is provided for opening or closing the passage through the second flow connection, wherein the second flow controller is arranged between the second branching point and the reservoir. The second flow controller can be configured, for example, as a valve or a clamp, e.g. as a shut-off valve or as a pinch valve. By means of the second flow controller the second flow connection can be closed between the second branching point and the reservoir during the second operational mode, so that the fluid cannot pass from the second branching point through the second flow connection. The fluid leaving the third flow connection is completely recycled to the filter unit. During the first operational mode, the second fluid controller opens the passage through the second flow connection, so that the fluid discharged from the filter unit can be recycled to the reservoir.

In particular in such embodiments comprising the first fluid controller and/or the second fluid controller it is possible, that the second pump is arranged in the third flow connection.

In other embodiments the third flow connection is configured without a flow controller, so that the third flow connection is always open during operation of the device. Configuring the third flow connection without a flow controller such as a valve or a clamp, considerably reduces the complexity of the device. The device becomes simpler and less expensive.

In still other embodiments the second flow connection is configured without a flow controller between the second branching point and the reservoir, so that the second flow connection is always open between the second branching point and the reservoir during operation of the device. Thus, it is possible to configure the device for tangential flow filtration without any flow controller (valve or clamp or the like). i.e. without a flow controller in the fist and the second and the third flow connection. This is particularly advantageous in view of minimizing the complexity and the cost of the device.

According to a preferred configuration the second pump is arranged between the second branching point and the second fluid opening of the filter unit.

Preferably, the first pump and the second pump are configured as centrifugal pumps.

Particularly preferred, at least one of the first and the second centrifugal pump comprises a rotor for conveying the fluid, and a stator which forms with the rotor an electromagnetic rotary drive for rotating the rotor about an axial direction, wherein the rotor comprises a magnetically effective core, and a plurality of vanes for conveying the fluid, wherein the stator is designed as a bearing and drive stator with which the rotor can be magnetically driven without contact and can be magnetically levitated without contact with respect to the stator. This embodiment of the centrifugal pump with a magnetically levitated rotor, which is simultaneously the pump rotor of the centrifugal pump and the rotor of the electromagnetic rotary drive for driving the rotation, enables an extremely compact, space-saving and powerful design of the first and/or second centrifugal pump. Due to the contactless magnetic levitation of the rotor, no mechanical bearings are required, which could lead to contamination of the fluid by abrasion, for example. The contactless magnetic levitation of the rotor also enables extremely precise and simple adjustment of the pressure generated by the first and the second centrifugal pump, for example via the rotational speed of the rotor.

Preferably, both centrifugal pumps comprise a rotor for conveying the fluid, and a stator which forms with the rotor an electromagnetic rotary drive for rotating the rotor about an axial direction, wherein the rotor comprises a magnetically effective core, and a plurality of vanes for conveying the fluid, wherein the stator is designed as a bearing and drive stator with which the rotor can be magnetically driven without contact and can be magnetically levitated without contact with respect to the stator.

With regard to the magnetic levitation of the rotor, it is particularly preferred that each rotor is in each case actively magnetically supported in a radial plane perpendicular to the axial direction and is passively magnetically stabilized in the axial direction and against tilting.

A particularly preferred embodiment is the embodiment as a temple motor, wherein each stator comprises a plurality of coil cores, each of which comprising a longitudinal limb extending in an axial direction and a transverse limb arranged in the radial plane and extending from the longitudinal limb in a radial direction, and wherein at least one concentrated winding is arranged on each longitudinal limb which surrounds the respective longitudinal limb. The embodiment as a temple motor is a particularly compact and simultaneously efficient embodiment.

According to a particularly preferred embodiment, each of the first pump and the second pump comprises in each case a pump unit having a pump housing, wherein the pump housing comprises an inlet and an outlet for the fluid to be conveyed, wherein the rotor is arranged in the pump housing and comprises the plurality of vanes for conveying the fluid, and wherein the pump unit is designed in such a way that the pump unit can be inserted into the stator, such that the rotor and the stator form the electromagnetic rotary drive for rotating the rotor about an axial direction. Preferably, the device according to the invention is configured in such a way that some components of the device, in particular those components which come into contact with the fluid, are configured as single-use parts which can be used only once and must be replaced after this use by new, i.e. unused, single-use parts.

For this reason, a set of single-use parts for a device according to the invention is proposed which set comprises at least the following components, each of which is configured as a single-use part:
- the filter unit;
- one pump unit in each case for each centrifugal pump,
- a plurality of tubes, which are configured for realizing the first flow connection, the second flow connection, and the third flow connection,
- and optionally a reservoir for the fluid or an insert for a reservoir.

It is understood that this list of components designed as single-use parts is not exhaustive. The set of single-use parts may also comprise other single-use parts, for example components of pressure sensors or flow sensors.

Furthermore, according to the invention, a method for tangential flow filtration of a fluid is proposed, comprising the steps of:
- providing a device for tangential flow filtration, which is configured according to claim 1,
- performing a first operational mode, in which the fluid is moved through the filter unit in a first flow direction from the first fluid opening to the second fluid opening of the filter unit,
- switching to a second operational mode, in which the fluid is moved through the filter unit in a second flow direction from the second fluid opening to the first fluid opening of the filter unit,
wherein at least in the second operational mode a partial flow of the fluid passes through the third flow connection.

An advantage of this operation using alternately the first operational mode and the second operational mode is that the filter element is alternately overflowed in opposite directions, namely in the first flow direction and in the second flow direction, whereby deposits on the filter element or the build-up of an undesired filter cake on the filter element can be avoided more efficiently and for a long time of operation.

The method can be performed, for example, such that in the first operational mode a partial flow of the fluid passes through the third flow connection.

According to a preferred embodiment, the second pump is not operated during the first operational mode.

As an option, in the second operational mode the passage through the second flow connection is closed, so that the fluid is circulated only through the third flow connection and the filter unit, and cannot return to the reservoir.

The changing between the first operational mode and the second operational mode is preferably performed according to a predeterminable time scheme.

Further advantageous measures and embodiments of the invention result from the dependent claims.

In the following, the invention will be explained in more detail, both in terms of apparatus and process technology, on the basis of embodiments and on the basis of the drawing. The drawing shows
- Fig. 1:: a schematic representation of a first embodiment of a device for tangential flow filtration according to the invention,
- Fig. 2:: a schematic representation of the first embodiment in a first operational mode,
- Fig. 3:: a schematic representation of the first embodiment in a second operational mode,
- Fig. 4:: a schematic representation of a second embodiment of a device for tangential flow filtration according to the invention,
- Fig. 5:: a schematic representation of the second embodiment in the first operational mode,
- Fig. 6:: a schematic representation of the second embodiment in the second operational mode,
- Fig. 7:: a schematic representation of a third embodiment of a device for tangential flow filtration according to the invention,
- Fig. 8:: a schematic representation of the third embodiment in the first operational mode,
- Fig. 9:: a schematic representation of the third embodiment in the second operational mode,
- Fig. 10:: a cross-sectional view of an embodiment of a centrifugal pump having a rotor, which can be magnetically contactlessly levitated, in a section in the axial direction, and
- Fig. 11:: an embodiment of a reservoir in a schematic representation.

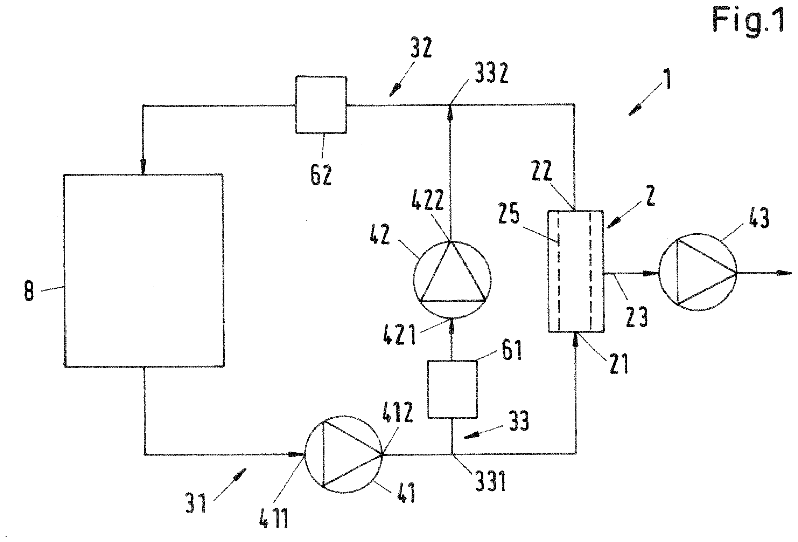

Fig. 1 shows in a schematic representation a first embodiment of a device for tangential flow filtration of a fluid according to the invention, which is designated in its entirety with the reference numeral 1. The fluid is, for example, a suspension such as a cell culture or a cell broth, from which at least one component shall be filtered out. Here, it is possible that the component to be filtered out, which is discharged as permeate, is the target product, which is to be recovered during filtration, for example a protein, or that by filtering out the permeate the fluid remaining as retentate is the target product, which is to be concentrated by filtration, so that, for example, the concentration of one or more components of the fluid increases.

The device 1 for tangential flow filtration comprises a filter unit 2 in which a filter element 25 is arranged, a first flow connection 31, with which the filter unit 2 can be connected to a reservoir 8 for the fluid, and a second flow connection 32, with which the filter unit 2 can be connected to the reservoir 8.

The filter unit 2 comprises a first fluid opening 21 and a second fluid opening 22 for the fluid, as well as a permeate opening 23 for discharging the permeate filtered out of the fluid.

The reservoir 8 is, for example, a bioreactor, in particular a perfusion bioreactor, for a perfusion process for the continuous cultivation of cells, whereby the filter unit 2 is used to filter out metabolic products of the cells or to obtain cell-free media, and the cells, or the suspension containing the cells, are subsequently returned to the bioreactor.

A first pump 41, preferably a centrifugal pump 41, for the fluid is arranged in the first flow connection 31, wherein the first centrifugal pump 41 has an inlet 411 and an outlet 412 for the fluid. The outlet 412 is connected to the first fluid opening 21 of the filter unit 2.

The second fluid opening 22 of the filter unit 2 is connected to the second flow connection 32, so that the fluid discharged from the filter unit 2 as retentate may be recirculated to the reservoir 8.

A third flow connection 33 is provided, which is configured for a fluid communication between the first flow connection 31 and the second flow connection 32, wherein the third flow connection 33 bypasses the filter unit 2. The third flow connection 33 branches off the first flow connection 31 at a first branching point 331 and is connected to the second flow connection 32 at a second branching point 332. The first branching point 331 is arranged downstream of the outlet 412 of the first pump 41 and upstream of the first fluid opening 21. The second branching point 332 is arranged in the second flow connection 32 between the second fluid opening 22 of the filter unit 2 and the reservoir 8.

A first flow controller 61 is provided in or at the third flow connection 33 and configured to open or to close the passage through the third flow connection 33. The first flow controller 61 can be switched by a control unit (not shown) between an open position, in which the passage through the third flow connection 33 is open, so that the fluid can pass through the third flow connection 33, and a closed position, in which the passage through the third flow connection 33 is closed, so that the fluid cannot pass through the third flow connection 33.

Furthermore, a second pump 42, preferably a centrifugal pump 42, for the fluid is arranged in the third flow connection 33. The second pump 42 may be arranged at any location in the third flow connection 33, for example between the first flow controller 61 and the second branching point 332, where the third flow connection 33 ends in the second flow connection 32. The second centrifugal pump 42 has an inlet 421 and an outlet 422 for the fluid. The outlet 422 is connected to the second branching point 332.

A second flow controller 62 is provided in or at the second flow connection 32 and configured to open or to close the passage through the second flow connection 32 at a location which is arranged between the reservoir 8 and the second branching point 332, where the third flow connection 33 ends in the second flow connection 32. The second flow controller 62 can be switched by a control unit (not shown) between an open position, in which the passage through the second flow connection 32 to the reservoir 8 is open, so that the fluid can pass through the second flow connection 32 to the reservoir 8, and a closed position, in which the passage through the second flow connection 32 to the reservoir 8 is closed, so that the fluid cannot pass through the second flow connection 32 to the reservoir 8.

Each of the first and the second flow controller 61, 62 can be configured as a valve or a clamp, e.g. as a shut-off valve or as a pinch valve.

A third pump 43, preferably a peristaltic pump 43, may by connected to the permeate opening 23 of the filter unit 2 for discharging the permeate from the filter unit 2.

The filter unit 2 is designed for a tangential flow filtration, which is also designated as cross-flow filtration. This means that the fluid in the filter unit 2 is guided parallel to the filter element 25, so that the fluid flows over the filter element 25, and the filtering out of the permeate takes place perpendicular to the flow direction of the fluid.

Such filter units 2, which are designed for tangential flow filtration, are sufficiently known to the person skilled in the art and therefore require no further explanation.

The first flow connection 31, the second flow connection 32 and the third flow connection 33 are preferably realized with pipes that are designed as flexible pipes, i.e., pipes whose walls can be deformed. Each pipe is designed, for example, as a tube, in particular as a plastic tube, made for example of a silicone rubber, PVC (polyvinyl chloride), PU (polyurethane), PE (polyethylene), HDPE (high density polyethylene), PP (polypropylene), EVA (ethyl vinyl acetate) or nylon. Preferably, each tube which belongs to the first flow connection 31 or the second flow connection 32 or the third flow connection 33 is designed for single use. When designed for single use, those components which come into contact with the substances to be treated, i.e. in this case in particular the tubes, are only used exactly once and are then replaced by new, i.e. unused, single-use parts for the next application.

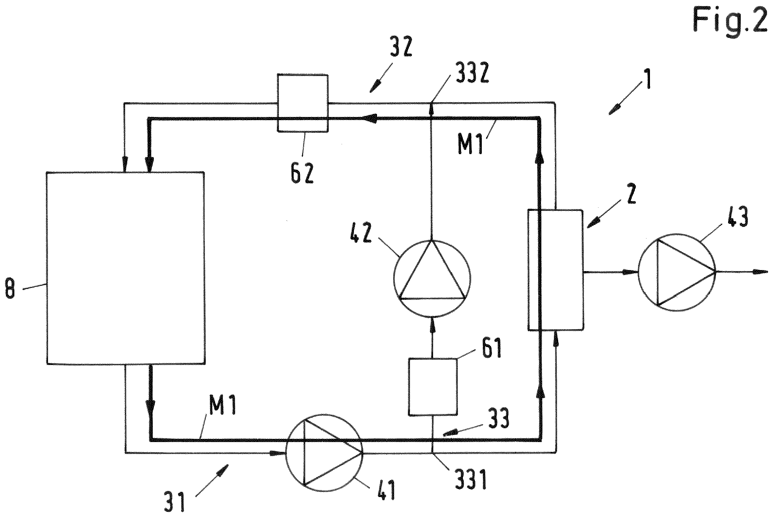

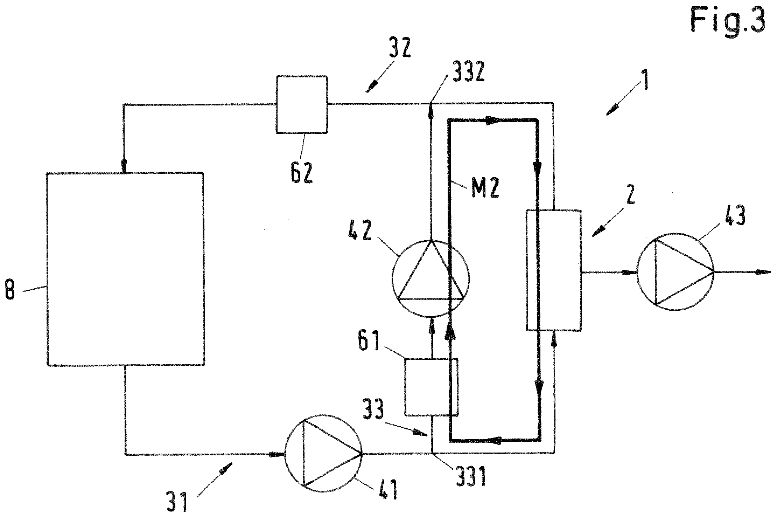

The device 1 can be operated in two different operational modes. Fig. 2 illustrates the first operational mode and Fig. 3 illustrates the second operational mode.

In the first operational mode the first flow controller 61 is in the closed position, so that the fluid cannot pass through the third flow connection 33. The first pump 41 is running and the second pump 42 is not running. The second flow controller 62 is in the open position, so that the fluid can pass through the second flow connection 32 to the reservoir 8. The flow of the fluid is indicated by the arrow with the reference numeral M 1. The first operational mode corresponds to the known operation of a device for tangential flow filtration. The fluid is moved through the filter unit 2 in a first flow direction from the first fluid opening 21 to the second fluid opening 22.

In the second operational mode a purging of the filter element 25 takes place by reversing the flow direction of the fluid through the filter unit 2. In the second operational mode the first flow controller 61 is in the open position, so that the fluid can pass through the third flow connection 33. The first pump 41 is running and the second pump 42 is also running. The second flow controller 62 is in the closed position, so that the fluid cannot pass through the second flow connection 32 to the reservoir 8. The flow of the fluid is indicated by the arrow with the reference numeral M2. The fluid is circulated by the second pump 42 through the third flow connection 33 to the second branching point 332 and from there to the second fluid opening 22 of the filter unit 2. The fluid leaves the filter unit 2 through the first fluid opening 21 and is then guided into the third flow connection 33 and back to the second pump 42. In the second operational mode the fluid is moved through the filter unit 2 in a second flow direction from the second fluid opening 22 to the first fluid opening 21.

Basically, all kinds of pumps can be used as first pump 41 and as second pump 42. However, it is preferred that the first pump 41 and the second pump 42 are each configured as centrifugal pumps. Particularly preferred, each of the first pump 41 and the second pump 42 is configured and operated or controlled, respectively, as it is disclosed for example in EP 4 101 520 A1.

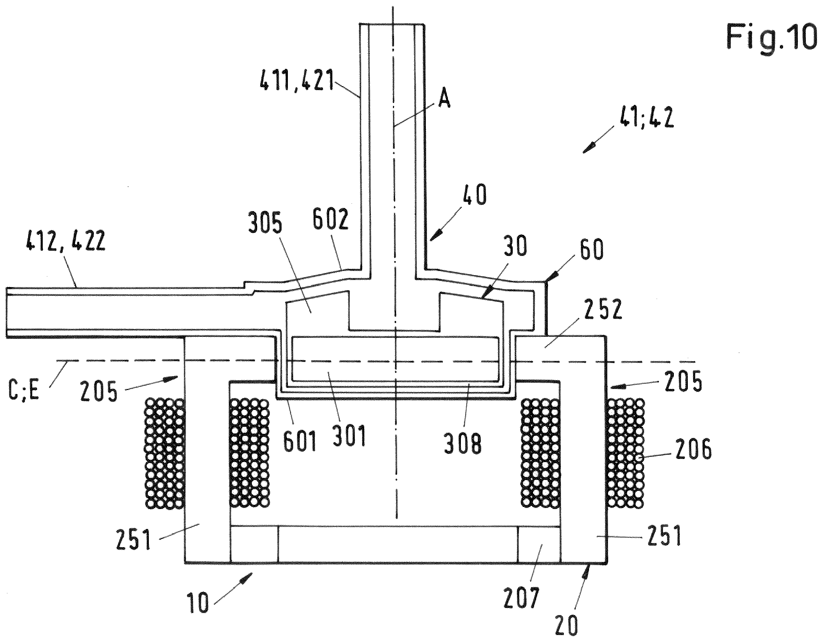

Fig. 10 shows a cross-sectional view of an embodiment of a centrifugal pump as it is preferred for the first pump 41 and the second pump 42. The section is in an axial direction A, which is defined by the nominal axis of rotation of the centrifugal pump 41, 42.

Since preferably both the first pump 41 and the second pump 42 are designed according to the embodiment illustrated in Fig. 10, in the following description no further linguistic distinction is made between the first centrifugal pump 41 and the second centrifugal pump 42, but reference is made to the centrifugal pump 41, 42, wherein the explanations then apply in the same way to the first pump 41 and to the second pump 42.

Particularly preferably, but not necessarily, the first centrifugal pump 41 and the second centrifugal pump 42 are designed, at least substantially, identically.

The centrifugal pump 41, 42 described in the following comprises a rotor 30 for conveying the fluid, and a stator 20 which forms with the rotor 30 an electromagnetic rotary drive 10 for rotating the rotor 30 about the axial direction A, wherein the rotor 30 comprises a magnetically effective core 301, and a plurality of vanes 305 for conveying the fluid, wherein the stator 20 is designed as a bearing and drive stator with which the rotor 30 can be contactlessly magnetically driven and can be contactlessly magnetically levitated with respect to the stator 20.

A particular advantage of this embodiment of the centrifugal pump 41, 42 is that the rotor 30 is designed as an integral rotor, because it is both the rotor 30 of the electromagnetic rotary drive 10 and the rotor 30 of the centrifugal pump 41, 42, with which the fluid is conveyed. In total, the rotor 30 thus fulfills three functions in one: It is the rotor 30 of the electromagnetic drive 10, it is the rotor 30 of the magnetic levitation, and it is the impeller with which the fluid is acted upon. This embodiment as an integral rotor offers the advantage of a very compact and space-saving design.

A further advantage is the contactless magnetic levitation of the rotor 30 with respect to the stator 20, which, due to the absence of mechanical bearings for the rotor 30, ensures that no contaminants, such as might occur in mechanical bearings, enter the fluid. In addition, due to the absence of mechanical bearings and the frictional forces occurring in them, the relationship between the electrical operating variables, such as drive current or drive voltage, and the rotational speed of the rotor 30 is much more precisely defined, which improves or simplifies the regulation of the centrifugal pump 41, 42.

The electromagnetic rotary drive 10 is designed according to the principle of the bearingless motor and is operated according to this principle. The term 'bearingless motor' means an electromagnetic rotary drive 10 in which the rotor 30 is levitated completely magnetically with respect to the stator 20, wherein no separate magnetic bearings are provided. For this purpose, the stator 20 is designed as a bearing and drive stator, which is both the stator 20 of the electric drive and the stator of the magnetic levitation. The stator 20 comprises electrical windings 206, with which a magnetic rotating field can be generated, which on the one hand exerts a torque on the rotor 30, which effects its rotation about the nominal axis of rotation defining the axial direction A, and which, on the other hand, exerts a transversal, i.e. radial, force onto the rotor 30, which can be adjusted as desired, so that its radial position can be actively controlled or regulated. Thus, three degrees of freedom of the rotor 30 can be actively controlled, namely its rotation and its radial position (two degrees of freedom). With respect to three further degrees of freedom, namely its position in the axial direction A and tilting with respect to the radial plane perpendicular to the nominal axis of rotation A (two degrees of freedom), the rotor 30 is preferably passively magnetically levitated or stabilized by reluctance forces, i.e. it cannot be controlled. The absence of a separate magnetic bearing with a complete magnetic levitation of the rotor 30 is the property, which gives the bearingless motor its name. In the bearing and drive stator, the bearing function cannot be separated from the drive function.

Regarding further detail of the bearingless motor reference is made for example to EP 4 101 520 A1

The nominal axis of rotation A refers to that axis about which the rotor 30 rotates in the operating state when the rotor 30 is in a centered and not tilted position with respect to the stator 20 as represented in Fig. 10. This nominal axis of rotation defines the axial direction A. Usually, the nominal axis of rotation defining the axial direction A corresponds to the central axis of the stator 20.

A radial direction refers to a direction, which stands perpendicular on the axial direction A.

The rotor 30 comprises the magnetically effective core 301, which is designed in a ring-shaped or disk-shaped manner. The magnetically effective core 301 is designed as a permanent magnetic disk or ring. That plane in which the magnetically effective core 301 of the rotor 30 is levitated in the stator 20 in the operating state is also referred to as the radial plane E. The radial plane defines the x-y plane of a Cartesian coordinate system whose z-axis extends in the axial direction A.

The radial position of the magnetically effective core 301 or the rotor 30 refers to the position of the rotor 30 in the radial plane E.

The "magnetically effective core 301" of the rotor 30 refers to that region of the rotor 30 which magnetically interacts with the stator 20 for torque generation and the generation of magnetic levitation forces.

The electromagnetic rotary drive 10 is designed as a temple motor and comprises the stator 20, which has a plurality of coil cores 205 - for example six coil cores 205 - each of which comprises a longitudinal limb 251 extending in the axial direction A, and a transverse limb 252, which is arranged perpendicular to the longitudinal limb 251 and which extends in a radial direction and is bounded by an end face. The coil cores 205 are arranged equidistantly on a circular line so that the end faces of the transverse limbs 252 surround the magnetically effective core 301 of the rotor 30. A concentrated winding 206 is arranged on each longitudinal limb 251, surrounding the respective longitudinal limb 251.

The longitudinal limbs 251 of the coil cores 205, which are aligned parallel to each other, and which all extend parallel to the axial direction A, and which surround the rotor 30 (or are surrounded by the rotor 30 in a design as an external rotor) are what gave the temple motor its name, because these parallel longitudinal limbs 251 are reminiscent of the columns of a temple.

Those ends of the longitudinal limbs 251 which face away from the transverse limbs 252 - in the representation of Fig. 10 these are the lower ends - are connected to each other by a flux guide 207. The flux guide 207 is preferably designed in a ring-shaped manner or comprises several segments that connect the longitudinal limbs 251 to one another. Both the flux guide 207 and the coil cores 205 of the stator 20 are each made of a soft magnetic material because they serve as flux conducting elements to guide the magnetic flux. Suitable soft magnetic materials for the coil cores 205 and the return 207 are, for example, ferromagnetic or ferrimagnetic materials, i.e., in particular iron, nickel-iron, cobalt-iron, silicon iron or Mu-metal. In this case, for the stator 20, a design as a stator sheet stack is preferred, in which the coil cores 205 and the return 207 are designed in sheet metal, i.e., they consist of several thin sheet metal elements, which are stacked.

The centrifugal pump 41, 42 comprises a pump unit 40 having a pump housing 60 which comprises the inlet 411; 421 and the outlet 412; 422 for the fluid to be conveyed, wherein the rotor 30 is arranged in the pump housing 60 and comprises a plurality of vanes 305 for conveying the fluid. The pump unit 40 is designed in such a way that the pump unit 40 can be inserted into the stator 20 such that the magnetically effective core 301 of the rotor 30 is surrounded by the end faces of the transverse limbs 252.

The pump housing 60 of the pump unit 40 comprises a base part 601 and a cover 602, which are connected to each other in a sealing manner, wherein the outlet 412; 422 is preferably, but not necessarily, completely arranged in the base part 601 of the pump housing. The cover 602 comprises the inlet 411; 412, which extends in the axial direction A, so that the fluid flows to the rotor 30 from the axial direction A.

The rotor 30 comprises the plurality of vanes 305 for conveying the fluid, for example a total of four vanes 305, whereby this number has an exemplary character. The rotor 30 further comprises a jacket 308 with which the magnetically effective core 301 of the rotor 30 is enclosed and preferably hermetically encapsulated so that the magnetically effective core 301 of the rotor 30 does not come into contact with the fluid to be conveyed. All vanes 305 are arranged on the jacket 308 and arranged equidistantly with respect to the circumferential direction of the rotor 30. Each vane 305 extends outward in the radial direction and is connected to the jacket 308 in a torque-proof manner. The vanes 305 may be separate components that are then fixed to the jacket 308. Of course, it is also possible that all of the vanes 305 are an integral part of the jacket 308, i.e., that the jacket 308 is designed with all of the vanes 305 as a single piece. The rotor 30 with the vanes 305 forms the vane or the impeller, respectively, of the centrifugal pump 41, 42, with which the fluid or fluids are acted upon.

The design of the centrifugal pump 41, 42 with the electromagnetic rotary drive 10 according to the principle of the bearingless motor also allows that the rotor 30 can be separated from the stator 20 very easily. This is a very great advantage, because in this way, for example, the rotor 30 or the pump unit 40 comprising the rotor can be designed as a single-use part for single use. Today, such single-use applications often replace processes in which, due to the very high purity requirements, all those components that come into contact with the substances to be treated in the process previously had to be cleaned and sterilized in an elaborate manner, for example by means of steam sterilization. When designed for single use, those components that come into contact with the substances to be treated are only used exactly once and are then replaced with new, i.e., unused, single-use parts for the next application.

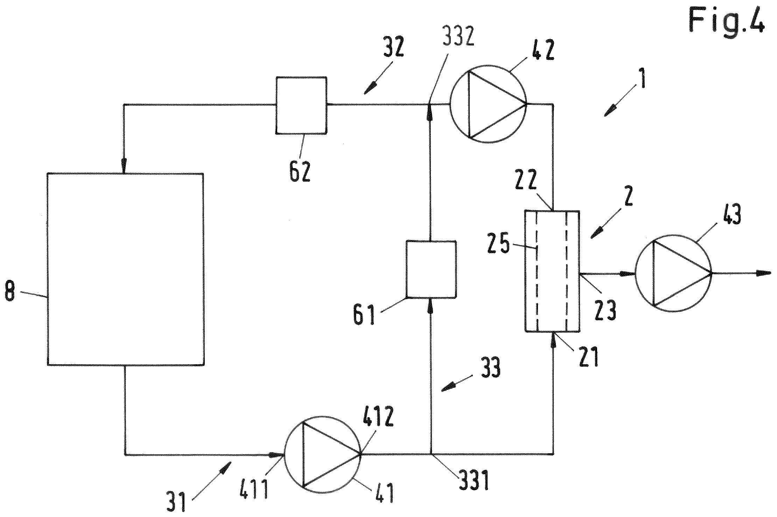

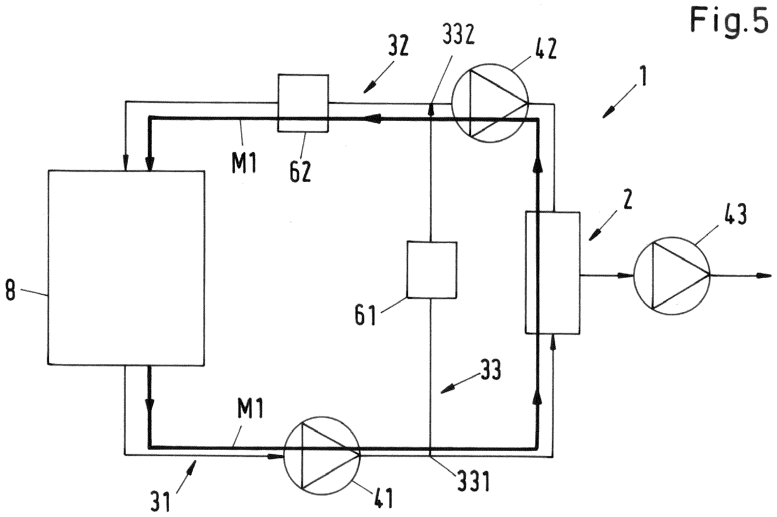

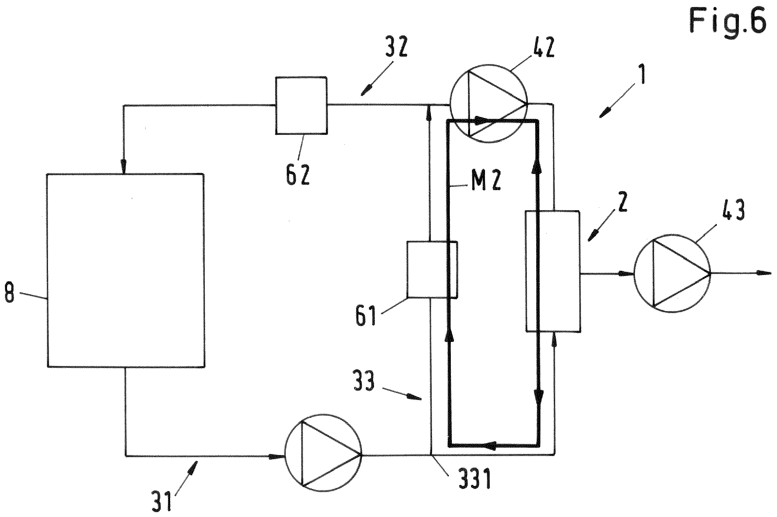

Fig. 4 shows a schematic representation of a second embodiment of a device for tangential flow filtration according to the invention. Fig. 5 shows a schematic representation of the second embodiment in the first operational mode, and Fig. 6 shows a schematic representation of the second embodiment in the second operating mode.

In the following description of the second embodiment, only the differences to the first embodiment will be discussed in more detail. Same parts or parts equivalent in function of the second embodiment are designated with the same reference numerals as in the first embodiment. In particular, the reference numerals have the same meaning as already explained in connection with the first embodiment. It has to be understood that the preceding explanations with respect to the first embodiment also apply to the second embodiment in the same way or in an analogous way.

In the second embodiment, the second pump 42 is arranged in the second flow connection 32 between the second branching point 332, where the third flow connection 33 ends in the second flow connection 32 and the second fluid opening 22 of the filter unit 2.

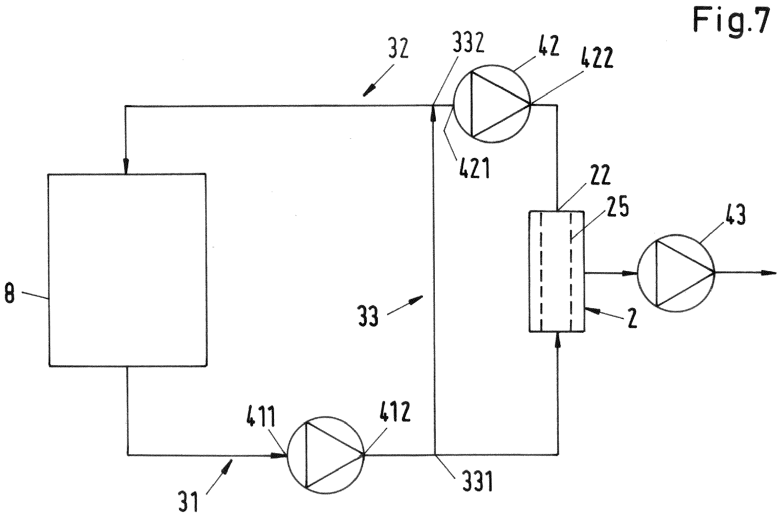

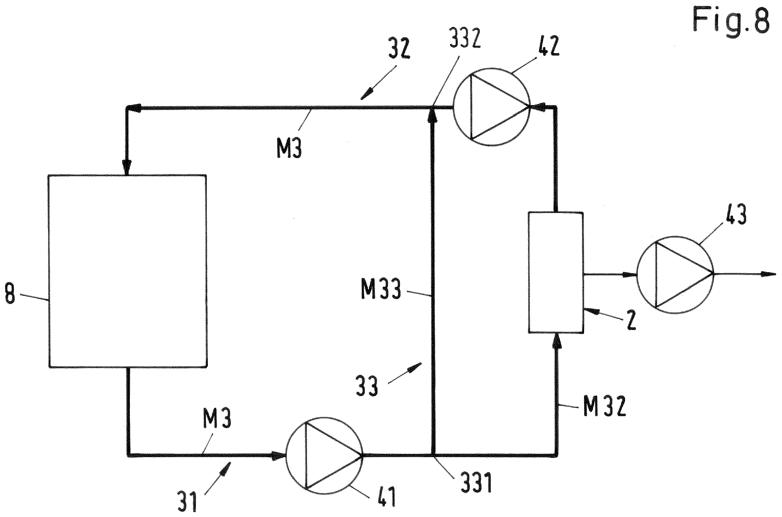

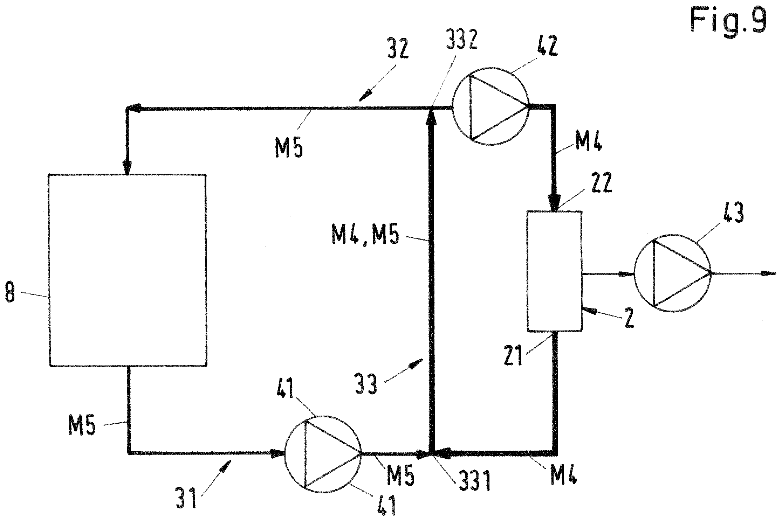

Fig. 7 shows a schematic representation of a third embodiment of a device 1 for tangential flow filtration according to the invention. Fig. 8 shows a schematic representation of the third embodiment in the first operational mode, and Fig. 9 shows a schematic representation of the third embodiment in the second operating mode.

In the following description of the third embodiment, only the differences to the first and the second embodiment will be discussed in more detail. Same parts or parts equivalent in function of the third embodiment are designated with the same reference numerals as in the first and the second embodiment. In particular, the reference numerals have the same meaning as already explained in connection with the first and the second embodiment. It has to be understood that the preceding explanations with respect to the first and the second embodiment also apply to the third embodiment in the same way or in an analogous way.

Compared to the first and the second embodiment, it is an essential difference that the third embodiment of the device 1 does neither comprise the first flow controller 61 nor the second flow controller 62.

The third flow connection 33 is configured without a flow controller, so that the passage through the third flow connection 33 is always open, both in the first operational mode (Fig. 8) and in the second operational mode (Fig. 9).

Furthermore, the second flow connection 32 is configured without a flow controller between the second branching point 332 and the reservoir 8 so that the passage from the second branching point 332 to the reservoir 8 through the second flow connection 32 is always open, both in the first operational mode (Fig. 8) and in the second operational mode (Fig. 9).

During the first operational mode (Fig. 8) the first pump 41 is operating and the second pump 42 is not operating. The flow of the fluid is indicated by the arrow with the reference numeral M3. The fluid is circulated by the first pump 41. At the first branching point 331 the fluid splits in a partial flow M33 passing through the third flow connection 33 to the second branching point 332, and a remaining flow M32 passing through the filter unit 2. The remaining flow M32 passes from the first branching point 331 to the first fluid opening 21 of the filter unit 2. The fluid leaves the filter unit 2 through the second fluid opening 22 and is then guided to the second branching point 332. At the second branching point 332 the partial flow M33 and the remaining flow M32 rejoin each other and are guided through the second flow connection 32 back into the reservoir 8.

The ratio of the partial flow M33 and the remaining flow M32 may be adjusted, for example, by the flow resistance of the third flow connection 33.

In the second operational mode (Fig. 9) the first pump 41 and the second pump 42 are both operational.

The flow of the fluid, generated by the second pump 42 is indicated by the arrows with the reference numeral M4.

The second pump 42 forces the flow of the fluid through the filter unit 2 in the second flow direction, i.e. from the second fluid opening 22 to the first fluid opening 21 of the filter unit 2. From there the flow M4 is guided to the first branching point 331. Since the first pump 41 is also operating, the flow M4 is forced into the third flow connection 33 together with a flow M5 of the fluid, which is generated by the first pump 41. Thus, both the flow M4 and the flow M5 pass from the first branching point 331 through the third flow connection 33 to the second branching point 332. At the second branching point 332 the flow splits in two directions. One part as indicated by the arrow M5 flows back to the reservoir 8 and the other part is guided to the second pump 42.

The first pump 41 and the second pump 42 may be operated with essentially the same rotational speed. However, it is preferred that the second pump 2 is operated with a rotational speed which is at least slightly smaller than the rotational speed, with which the first pump 41 is operated.

Furthermore, the second pump 42 is controlled such, that the pressure of the flow M4 at the first branching point 331 does not exceed the pressure of the flow M5 generated by the first pump 41 at the first branching point 331. Therewith, a reverse flow of the fluid through the outlet 412 into the first pump 41 can be reliably avoided.

It is also possible that the device 1 according to the invention further comprises the reservoir 8. Such embodiments are possible, in which the entire reservoir 8 is designed as a single-use part, for example as a dimensionally stable plastic container, and such embodiments, in which only one component of the reservoir 8 is designed as a single-use part.

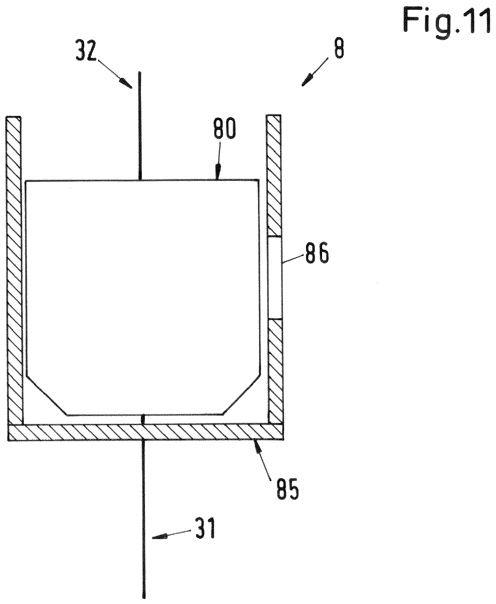

Such an embodiment of the reservoir 8 is represented in a schematic representation in Fig. 11. The reservoir 8 comprises a flexible insert 80 for receiving the fluid, which is made of a plastic. The insert 80 is preferably a flexible bag, for example a plastic bag or a synthetic bag, which can be folded so that it requires as little space as possible during storage. The insert 80 may comprise additional inlets or outlets (not shown), for example for supplying additional substances, e.g., nutrient solutions or gases such as oxygen. It is also possible to use a further inlet for receiving probes or measurement sensors with which parameters are monitored, e.g., temperature, pressure, concentrations, etc. The inlet(s) can also be for mass transfer. In particular, necessary gases can be supplied or discharged here, for example in an embodiment as bioreactor. In particular in the cultivation of microorganisms or biological cells, it is often a necessity that oxygen or air can be supplied to the container 80 and other gases, in particular carbon dioxide, can be discharged from the container.

In particular, so-called sampling ports (not shown) can be glued or welded to the reservoir 8 or to the insert 80. These are short tube-like plastic structures through which, for example, samples can be taken from the insert 80. Each sampling port is usually secured by a clamp at its end protruding from the insert 80 in a manner known per se, so that no undesired substances can enter the interior of the insert 80 through these sampling ports.

The reservoir 8 further comprises a dimensionally stable or rigid support container 85, which is designed as a reusable component and for receiving the insert 80. At least one window 86 may be provided on the wall of the support container 85, through which a visual access to the insert 80 is possible.

In particular with regard to such embodiments of the device 1, which comprise reusable components for multiple use (e.g. the stator 20) as well as components for single use, a set of single-use parts for a device 1 according to the invention is further proposed, which comprises at least the following components, which are each configured as single-use parts: the filter unit 2, a pump unit 40 for each centrifugal pump 41, 42, a plurality of tubes, which are configured to realize the first flow connection 31, the second flow connection 32 and the third flow connection 33, and optionally a reservoir 8 for the fluid or an insert 80 for a reservoir 8.

## Claims

1. A device for tangential flow filtration of a fluid, comprising a filter unit (2) which has a first fluid opening (21) and a second fluid opening (22) for the fluid, as well as a filter element (25) and a permeate opening (23) for discharging a permeate filtered out of the fluid, wherein the device further comprises a first flow connection (31) by means of which the first fluid opening (21) can be connected to a reservoir (8) for the fluid, and a second flow connection (32) by means of which the second fluid opening (22) can be connected to the reservoir (8) for the fluid, wherein a first pump (41) is provided in the first flow connection (31), with which the fluid can be conveyed from the reservoir (8) to the filter unit (2), **characterized in that** a third flow connection (33) is provided, configured for a fluid communication between the first flow connection (31) and the second flow connection (32), wherein the third flow connection (33) bypasses the filter unit (2), and wherein a second pump (42) is provided for circulating the fluid through the third flow connection (33) and the filter unit (2).

2. A device according to claim 1, comprising a first branching point (331) arranged at the first flow connection (31), and a second branching point (332) arranged at the second flow connection (32), wherein the third flow connections (33) extends from the first branching point (331) to the second branching point (332), and wherein the first branching point (331) is arranged between the first pump (41) and the first fluid opening (21) of the filter unit (2)

3. A device according to anyone of the preceding claims, wherein a first flow controller (61) is provided for opening or closing the passage through the third flow connection (33).

4. A device according to anyone of claims 2-3, wherein a second flow controller (62) is provided for opening or closing the passage through the second flow connection (32), wherein the second flow controller (62) is arranged between the second branching point (332) and the reservoir (8).

5. A device according to anyone of the preceding claims, wherein the second pump (42) is arranged in the third fluid communication (33).

6. A device according to anyone of claims 1-2, wherein the third flow connection (33) is configured without a flow controller, so that the third flow connection (33) is always open during operation of the device.

7. A device according to claim 6, wherein the second flow connection (32) is configured without a flow controller between the second branching point (332) and the reservoir (8), so that the second flow connection (32) is always open between the second branching point (332) and the reservoir (8) during operation of the device.

8. A device according to anyone of claims 2-7, wherein the second pump (42) is arranged between the second branching point (332) and the second fluid opening (22) of the filter unit.

9. A device according to anyone of the preceding claims, wherein each of the first pump (41) and the second pump (42) comprises in each case a pump unit (40) having a pump housing (60), wherein the pump housing (60) comprises an inlet (411; 421) and an outlet (412; 422) for the fluid to be conveyed, wherein a rotor (30) is arranged in the pump housing (60) and comprises a plurality of vanes (305) for conveying the fluid, and wherein the pump unit (40) is designed in such a way that the pump unit (40) can be inserted into a stator (20), such that the rotor (30) and the stator (20) form an electromagnetic rotary drive (10) for rotating the rotor about an axial direction (A).

10. A set of single-use parts for a device according to claim 9, which comprises at least the following components, each of which is configured as a single-use part:
- the filter unit (2);
- one pump unit (40) in each case for each centrifugal pump (41, 42),
- a plurality of tubes, which are configured for realizing - the first flow connection (31), the second flow connection (32), and the third flow connection (33),
- and optionally a reservoir (8) for the fluid or an insert (80) for a reservoir (8).

11. A method for tangential flow filtration of a fluid, comprising the steps of:
- providing a device for tangential flow filtration, which is configured according to claim 1,
- performing a first operational mode, in which the fluid is moved through the filter unit (2) in a first flow direction from the first fluid opening (21) to the second fluid opening (22) of the filter unit (2),
- switching to a second operational mode, in which the fluid is moved through the filter unit (2) in a second flow direction from the second fluid opening (22) to the first fluid opening (21) of the filter unit (2),
**characterized in that** at least in the second operational mode a partial flow of the fluid passes through the third flow connection (33).

12. A method according to claim 11, wherein in the first operational mode a partial flow of the fluid passes through the third flow connection (33).

13. A method according to anyone of claims 11-12, wherein the second pump (42) is not operated during the first operational mode.

14. A method according to claim 11, wherein in the second operational mode the passage through the second flow connection (32) is closed, so that the fluid is circulated only through the third flow connection (33) and the filter unit (2), and cannot return to the reservoir (8).

15. A method according to anyone of claims 11-14, wherein changing between the first operational mode and the second operational mode is performed according to a predeterminable time scheme.
